# EUROPÄISCHE PATENTSCHRIFT

(11) **EP 1 239 853 B1**
(45) Veröffentlichungstag und Bekanntmachung des Hinweises auf die Patenterteilung: **08.11.2006**
(21) Anmeldenummer: 01965273.4
(22) Anmeldetag: 10.09.2001
(51) Int. Cl.: A61K 31/185, A61P 11/02

(54) **MITTEL ENTHALTEND N-CHLORTAURIN SOWIE DESSEN VERWENDUNG ZUR BEHANDLUNG VON SCHLEIMHAUTPOLYPEN**
AGENT CONTAINING N-CHLOROTAURINE AND ITS USE FOR THE TREATMENT OF MUCOSAL POLYPS
PRODUIT CONTENANT DE LA N-CHLOROTAURINE ET SON UTILISATION POUR LE TRAITMENT DE POLYPES MUCOSALES

(30) Priorität: 14.09.2000 DE 10045868
(43) Veröffentlichungstag der Anmeldung: 18.09.2002
(73) Patentinhaber: Gottardi, Waldemar, 6020 Innsbruck (AT)
(72) Erfinder: GOTTARDI, Waldemar, A-6020 Innsbruck (AT); NAGL, Markus, A-6094 Axams (AT); NEHER, Andreas, A-6020 Innsbruck (AT)
(74) Vertreter: Harders, Gerhard
(86) Internationale Anmeldenummer: PCT/EP2001/010437
(87) Internationale Veröffentlichungsnummer: WO 2002/022118

(56) Entgegenhaltungen:
- DE-A- 4 041 703
- DE-A- 19 712 565
- NAGL MARKUS ET AL: "Enhanced fungicidal activity of N-chlorotaurine in nasal secretion." JOURNAL OF ANTIMICROBIAL CHEMOTHERAPY, Bd. 47, Nr. 6, Juni 2001 (2001-06), Seiten 871-874, XP001027190 ISSN: 0305-7453
- NAGL MARKUS ET AL: "Enhancement of the bactericidal efficacy of N-Chlorotaurine by inflammation samples and selected N-H compounds" HYG MED, Bd. 21, Nr. 11, 1996, Seiten 597-605, XP001055695
- NAGL MARKUS ET AL: "Tolerance of N-chlorotaurine, a new antimicrobial agent, in infectious conjunctivitis - A phase II pilot study." OPHTHALMOLOGICA, Bd. 214, Nr. 2, März 2000 (2000-03), Seiten 111-114, XP001027188 ISSN: 0030-3755
- NAGL MARKUS ET AL: "Tolerance and bactericidal action of N-chlorotaurine in a urinary tract infection by an omniresistant Pseudomonas aeruginosa." ZENTRALBLATT FUER BAKTERIOLOGIE, Bd. 288, Nr. 2, Oktober 1998 (1998-10), Seiten 217-223, XP001027177 ISSN: 0934-8840
- WAGNER D K ET AL: "INHIBITION OF NEUTROPHIL KILLING OF CANDIDA-ALBICANS PSEUDOHYPHAE BY SUBSTANCES WHICH QUENCH HYPOCHLOROUS-ACID AND CHLORAMINES" INFECTION AND IMMUNITY, Bd. 51, Nr. 3, 1986, Seiten 731-735, XP001027455 ISSN: 0019-9567
- DATABASE WPI Section Ch, Week 199540 Derwent Publications Ltd., London, GB; Class C03, AN 1995-308960 XP002188307 & JP 07 206609 A (SOGO YAKKO KK), 8. August 1995 (1995-08-08)
- NAGL MARKUS ET AL: "The postantibiotic effect of N-chlorotaurine on Staphylococcus aureus. Application in the mouse peritonitis model." JOURNAL OF ANTIMICROBIAL CHEMOTHERAPY, Bd. 43, Nr. 6, Juni 1999 (1999-06), Seiten 805-809, XP001027194 ISSN: 0305-7453

## Beschreibung

Die vorliegende Erfindung betrifft die Verwendung von N-Chlortaurin oder eines seiner Salze zur Herstellung eines Arzneimittels zur Behandlung von bei Rhinosinusitis auftretenden Schleimhautpolypen. Es wurde überraschenderweise gefunden, dass N-Chlortaurin (NCT), insbesondere in Form seiner Salze, wie z.B. des Natrium-Salzes (NCT-Na), bei der Behandlung von Schleimhautpolypen (Polyposis) ausgezeichnet wirkt.

NCT-Na wurde 1991 erstmals synthetisiert und hat sich inzwischen als ein vielseitig verwendbares neues Desinfektionsmittel in der Humanmedizin herausgestellt. Dies beruht einerseits auf seiner weitgestreuten mikrobiziden Wirkung, die gegenüber Bakterien und Viren *in vitro* bereits eingehend dokumentiert ist.

NCT-Na wird *in vivo* hervorragend vertragen, was am Auge, im Mittelohr, am äußeren Ohr, auf Hautgeschwüren, in der. Mundhöhle und der Harnblase nachgewiesen werden konnte.

Versuche z. B. mit Rhinosinusitis-Patienten zeigten, dass durch Behandlung der inneren Nasenoberflächen mit NCT-Na Lösung sich die Symptome dieser Krankheit, wie Schmerzen, Kopfdruck, Verlust des Geruchssinns, verstärkte Sekretion und Polypenbildung weitgehend gebessert haben und sogar Beschwerdefreiheit auftrat. Behandlungen mit herkömmlichenfungiziden Mitteln haben hingegen bisher zu keinem Erfolg geführt :

Die Bedeutung des Mittels liegt unter anderem darin, dass bei den bei Rhinosinusitis auftretenden Schleimhautpolypen eine Rückbildung beobachtet werden konnte, sodass die bisher erfolgreiche Standardtherapie, nämlich die operative Entfernung, durch den Einsatz des erfindungsgemäßen Mittels vermieden werden kann.

Aufgabe der vorliegenden Erfindung ist es, ein Mittel zu schaffen, das eine erfolgreiche Therapie von Polyposis unter schonenden Bedingungen und mit einem minimalen Risiko für den Patienten ermöglicht.

Gelöst wird diese Aufgabe erfindungsgemäß durch ein gattungsgemäßes Mittel mit den Merkmalen des kennzeichnenden Teils des Hauptanspruchs. Die Unteransprüche geben bevorzugte Ausgestaltungen der Erfindung wieder.

Das erfindungsgemäße Mittel zur Behandlung von durch Pilzinfektion verursachten Krankheiten kann auf folgende Weise auf die betroffenen äußeren und inneren Oberflächen appliziert werden:
- Spülungen;
- Inhalation;
- Versprühen;
- Aufpinseln
- oral (Verschlucken).

Als übliche Zusatzstoffe können dem erfindungsgemäßem Mittel u.a. beispielsweise folgende Stoffe zugegeben werden:
- Kochsalz, z.B. zum Einstellen einer isotonischen Lösung;
- Puffersubstanzen;
- Stabilisatoren;
- Geschmacks- und Geruchskorrigentien
- Konservierungsmittel.

Die Konzentration des N-Chlortaurin kann in weiten Grenzen schwanken und beispielsweise im Bereich von 0.001 bis 50% liegen, vorzugsweise im Bereich von 0.1 bis 10%, insbesondere 0.5 bis 2%. In der praktischen Anwendung hat sich eine Konzentration von ca. 1% als zweckmäßig erwiesen, insbesondere bei einer Behandlung durch Spülung oder Versprühen.

Die in dieser Anmeldung angegebenen %-Angaben beziehen sich, soweit nicht anders angegeben, auf Gew.-%.

Die Erfindung wird nachfolgend anhand de Beispiel näher erläutert:

### Beispiel

Eine 33-jährige Patientin litt seit ca. 1 Jahr an chronischer Rhinosinusitis mit Schleimhautschwellungen und vermehrter Sekretbildung in der Nasenhöhle und in sämtlichen Nasen-Nebenhöhlen, starker Polyposis sowie Verlust des Geruchssinns.

Die Behandlung mit 1% NCT-Na erfolgte über insgesamt 4 Wochen, 3x wöchentlich, und umfasste insgesamt 12 Behandlungen. Es wurden jeweils 10 mL NCT-Na Lösung mittels eines Nasenkatheters (Modell YAMIK) über einen Zeitraum von 12 min appliziert. Danach wurde die Spüllösung abgesaugt.

Behandlungsergebnis: Nach 6maliger Applikation kam es zu einer weitgehenden Regeneration des Geruchssinns. Am Ende der Behandlungen war eine eindeutige Rückbildung der Polyposis nasi festzustellen.

## Patentansprüche

1. Verwendung von N-Chlortaurin oder eines seiner Salze in wässriger Lösung, gegebenenfalls mit üblichen Zusatzstoffen, zur Herstellung eines Arzneimittels zur Behandlung von bei Rhinosinusitis auftretenden Schleimhautpolypen.

2. Verwendung nach Anspruch 1, **dadurch gekennzeichnet, daß** die wässrige N-Chlortaurinlösung eine Konzentration von 0,001 bis 50 % hat.

3. Verwendung nach Anspruch 2, **dadurch gekennzeichnet, daß** die wässrige N-Chlortaurinlösung eine Konzentration von 0,1 bis 10% hat.

4. Verwendung nach Anspruch 3, **dadurch gekennzeichnet, daß** die wässrige N-Chlortaurinlösung eine Konzentration von 0,5 bis 2 % hat.

5. Verwendung nach Anspruch 1, **dadurch gekennzeichnet, daß** N-Chlortaurin in Form eines Alkalisalzes eingesetzt wird.

6. Verwendung nach Anspruch 5, **dadurch gekennzeichnet, daß** N-Chlortaurin in Form des Natriumsalzes eingesetzt wird.

## Claims

1. Use of N-chlorotaurine or one of its salts in aqueous solutions, in given cases with customary additives, for the preparation of a pharmaceutical for treatment of mucus tissue polyps occuring in rhinosinusitis.

2. Use according to claim 1, **characterized in that** the aqueous solution of N-chlorotaurine has a concentration of 0,001 to 50 %.

3. Use according to claim 2, **characterized in that** the aqueous solution of N-chlorotaurine has a concentration of 0,1 to 10 %.

4. Use according to claim 3, **characterized in that** the aqueous solution of N-chlorotaurine has a concentration of 0,2 to 2 %.

5. Use according to claim 1, **characterized in that** the N-chlorotaurine is used in form of an alkali salt.

6. Use according to claim 5, **characterized in that** the N-chlorotaurine is used in form of the sodium salt.

## Revendications

1. Utilisation de N-chlortaurine ou d'un de ses sels dans une solution aqueuse, le cas échéant avec des substances habituelles, pour la fabrication d'un médicament pour le traitement des polypes muqueuses surgis dans rhinosinusite.

2. Utilisation selon exigence 1, **caractérisé en ce que** concentration de la solution aqueuse entre 0,001 et 50%.

3. Utilisation selon exigence 2, **caractérisé en ce que** concentration de la solution aqueuse entre 0,1 et 10%.

4. Utilisation selon exigence 3, **caractérisé en ce que** concentration de la solution aqueuse entre 0,5 et 2%.

5. Utilisation selon exigence 1, **caractérisé en ce que** N-Chlortaurin est utilisé comme sel alkali.

6. Utilisation selon exigence 5, **caractérisé en ce que** N-Chlortaurin est utilisé comme sel sodique.
